## Europäisches Patentamt

## European Patent Office

## Office européen des brevets

(11) Publication number: **0 329 761 B1**

## EUROPEAN PATENT SPECIFICATION

(45) Date of publication of patent specification: **01.12.93**  (51) Int. Cl.⁵: **C07K 7/08**, G01N 33/569

(21) Application number: **88907953.9**

(22) Date of filing: **19.08.88**

(86) International application number:
**PCT/US88/02870**

(87) International publication number:
**WO 89/01494 (23.02.89 89/05)**

(54) **HIV-1-RELATED POLYPEPTIDES, DIAGNOSTIC SYSTEMS AND ASSAY METHODS.**

(30) Priority: **21.08.87 US 88067**

(43) Date of publication of application:
**30.08.89 Bulletin 89/35**

(45) Publication of the grant of the patent:
**01.12.93 Bulletin 93/48**

(84) Designated Contracting States:
**AT BE CH DE FR GB IT LI LU NL SE**

(56) References cited:
**US-A- 4 629 783**

**ALIZON, Cell, 46, 63-74, published July 1986**

**GUYANDER, Nature, 326, 662-669, published 1987**

**GNANN, Science, 237, 7346-7349, published September 1987**

**GNANN, Journal of Virology, 61, 2639-2641, published August 1987**

**GNANN, The Journal of Infections Diseases, 156, 261-267, published August 1987**

(73) Proprietor: **SCRIPPS CLINIC AND RESEARCH FOUNDATION**
**10666 North Torrey Pines Road**
**La Jolla California 92037(US)**

(72) Inventor: **OLDSTONE, Michael**
**7710 Exchange Place**
**La Jolla, CA 92037(US)**
Inventor: **GNANN, John, W., Jr.**
**7222 Enders Avenue**
**San Diego, CA 92122(US)**

(74) Representative: **Fisher, Adrian John et al**
**CARPMAELS & RANSFORD**
**43 Bloomsbury Square**
**London WC1A 2RA (GB)**

**Description**

Technical Field

The present invention contemplates novel polypeptide antigens related to the human immunodeficiency virus type 1 (HIV-1) and to their use in diagnostic systems and assay methods.

Background

The acquired immune deficiency syndrome (AIDS) has now spread worldwide and appears to be an acute public health problem, particularly in Africa. A retrovirus designated human immunodeficiency virus type 1 (HIV-1), but previously known as LAV, HTLV-III or ARV, was shown to cause AIDS in the different areas afflicted by the epidemics, including North America, Western Europe and Central Africa.

Studies of HIV-1 at the molecular level have revealed some differences in the nucleotide sequence of North-American and African isolates. However, the North American, Western European and Central African isolates appear to have similar biological properties and antigenically cross-reactive proteins with the same relative molecular mass.

Individuals infected with HIV-1 develop antibodies to the gag-gene encoded viral core proteins designated p19, p24, and to their precursor protein designated p55. In addition, antibodies against env-gene encoded envelope glycoproteins gp120 (the extracellular glycoprotein or EGP), gp41 (the transmembrane protein or TMP) and their precursor glycoprotein designated gp160 are also observed.

A large number of AIDS patients show a disappearance of antibodies to the HIV-1 core proteins at an advanced state of the disease, but retain antibodies immunoreactive with the envelope antigens. The envelope products are regularly detected by antibodies in sera from patients at different stages of HIV-1 infection. Although few reports are available on seroconversion to HIV-1, the emergence of antibodies to the envelope proteins seems to shortly precede emergence of antibodies to the core proteins. See Carlson et al., Lancet, i:361-362, (1987). For these reasons, the use of antigens representing the env-gene products have significant importance in diagnosis of exposure to AIDS related retroviruses.

Because AIDS can be transmitted by blood products, there has, from the initial recognition of the disease, been a strong impetus to develop diagnostic tests to screen blood for antibodies or antigens specific for the infecting virus. Efforts in this area have borne fruit, and by the end of 1985 five companies had been approved to market tests to detect antibodies to HIV-1 virus. These tests all rely for detection of those antibodies on the use of viral proteins obtained from cultured HIV-infected T-lymphocytes. The virus obtained from the cultured cells is disrupted (e.g., with detergent) and a fluid (called "viral lysate") is obtained. This lysate (containing a variety of fragments of viral and cellular protein) is then typically used as the solid phase component of an immunoassay.

While the existing tests appear to have significantly diminished the transmission of HIV-1 via blood products, the viral lysate based tests have some significant disadvantages, including results that indicate a high rate of false positives. The false positives are thought to be due in part to the presence of non-viral proteins in the viral lysate preparations used in the solid phase component of the current assays.

In an attempt to reduce the rate of false positive results, the art has begun to develop site-directed serological assays employing synthetic polypeptides that mimic naturally occurring antigenic determinants on viral proteins. For instance, the United States Patent No. 4,629,783 to Cosand, Wang et al., Proc. Natl. Acad. Sci. USA, 83:6159-6163 (1986), and Kennedy et al., Science, 231:1556-1559 (1986) describe several polypeptides said to be capable of mimicking antigenic determinants formed by various HIV-1 proteins, including the TMP (gp41).

More specifically, the patent to Cosand describes a 26 amino acid residue polypeptide, designated polypeptide 39, whose sequence was derived from the TMP sequence of the HIV-1 isolate described by Wain-Hobson et al., Cell, 40:9-15 (1985). The Cosand patent contains data suggesting that polypeptide 39 can detect antibodies induced by HIV-1 TMP in sera of unidentified geographic origin.

Peptide number 8 reported by Wang et al., supra, contains 21 amino acid residues corresponding in sequence to TMP residues 586-606 of the HIV-1 sequence reported by Ratner et al., Nature, 313:277-284 (1985). According to Wang et al., polypeptide 8 can detect anti-HIV-1-TMP antibodies in sera collected from individuals within the United States. Furthermore, Wang et al. reported that analogs of polypeptide 8 containing conservative substitutions of valine for isoleucine at position 587 and arginine for lysine at position 596, as found in certain virus isolates, did not much affect the human antibody binding to those analogs.

More recently, Gnann et al., J. Infect. Dis., 156:261-267 (1987) reported that a polypeptide derived from the TMP amino acid residue sequence of Wiley et al., Proc. Natl. Acad. Sci. USA, 83:5038 (1986) can detect anti-HIV-1 TMP antibodies in the sera of virtually 100% of American AIDS patients but can detect those antibodies in only about 84% of Zairian AIDS patients.

In addition to their use as diagnostic reagents, Fischinger et al., Cancer Res., 45:46945-6995 (1985), have suggested that synthetic polypeptides can be used as vaccine components for HIV. This approach to AIDS prevention and therapy is supported by the report of Chanh et al., EMBO, 5:3065-71 (1986) wherein a polypeptide having an amino acid residue sequence corresponding to TMP residues 503 to 532 of the HIV-1 isolate sequence reported by Muesing et al., Nature, 313:450 (1985) was found to be capable of inducing HIV-neutralizing antibodies in rabbits. Gnann et al., J. Virol., 61:2639-41 (1987) report a similar result using a polypeptide corresponding to residues 598-609 of the HIV-1 isolate of Wain-Hobson et al., supra.

Brief Summary of the Invention

Two specific polypeptides having an improved ability to detect anti-HIV-1 antibodies in body samples from central Africans have been discovered. Thus, the present invention contemplates a polypeptide consisting essentially of 12 amino acid resides having a sequence represented by the formula:
LGZWGCSGKHIC,
wherein Z is either isoleucine or methionine.

Also contemplated is a composition comprising an admixture of at least two polypeptides wherein a first of the polypeptides has an amino acid residue sequence of the formula:
LGZWGCSGKHIC,
wherein Z is either isoleucine or methionine, and a second of the polypeptides is capable of immunoreacting with antibodies induced by a human immunodeficiency virus (HIV).

Further contemplated is a method of assaying for the presence of anti-HIV-1 antibodies in a body fluid sample. An immunoreaction admixture is formed by admixing a body fluid-sample with a polypeptide of the formula:
LGZWGCSGKHIC,
wherein Z is either isoleucine or methionine.

The immunoreaction admixture is maintained under biological assay conditions for a time period sufficient for any anti-HIV-1 antibodies present in the sample to immunoreact with the polypeptide and form a polypeptide-containing immunoreaction product.

The presence of any polypeptide-containing immunoreaction product that formed is then determined, and thereby the presence of any anti-HIV-1 antibodies in said sample.

In another embodiment a method of assaying for the presence of anti-HIV antibodies in a body fluid sample comprises forming an immunoreaction admixture by admixing a body fluid sample with a composition comprising an admixture of at least two polypeptides wherein a first of the polypeptides has an amino acid residue sequence represented by the formula:
LGZWGCSGKHIC,
wherein Z is either isoleucine or methionine, and a second of the polypeptides is either:
LGLWGCSGKLIC,
LGIWGCSGKLIC, or
NSWGCAFRQVC.

The immunoreaction admixture so formed is maintained under biological assay conditions for a time period sufficient for any anti-HIV antibodies present in the sample to immunoreact with a polypeptide of the composition and form a polypeptide-containing immunoreaction product.

The presence of any polypeptide-containing immunoreaction product that formed is then determined, and thereby the presence of any anti-HIV antibodies in the sample.

Also contemplated is a diagnostic system in kit form for assaying for the presence of anti-HIV-1 antibodies in a body fluid sample comprising a package containing a polypeptide of the formula:
LGZWGCSGKHIC,
wherein Z is either isoleucine or methionine.

In another embodiment, a diagnostic system in kit form for assaying for the presence of anti-HIV antibodies in a body fluid sample comprises a package containing at least two polypeptides wherein a first of the polypeptides has the formula:
LGZWGCSGKHIC,
wherein Z is either isoleucine or methionine;
and a second of the polypeptides is either

LGLWGCSGKLIC,
LGIWGCSGKLIC, or
NSWGCAFRQVC.

Detailed Description of the Invention

A. Definitions

Amino Acid: All amino acid residues identified herein are in the natural L-configuration. In keeping with standard polypeptide nomenclature, J. Biol. Chem., 243:3557-59, (1969), abbreviations for amino acid residues are as shown in the following Table of Correspondence:

| TABLE OF CORRESPONDENCE | | |
| --- | --- | --- |
| SYMBOL | | AMINO ACID |
| 1-Letter | 3-Letter | |
| Y | Tyr | L-tyrosine |
| G | Gly | glycine |
| F | Phe | L-phenylalanine |
| M | Met | L-methionine |
| A | Ala | L-alanine |
| S | Ser | L-serine |
| I | Ile | L-isoleucine |
| L | Leu | L-leucine |
| T | Thr | L-threonine |
| V | Val | L-valine |
| P | Pro | L-proline |
| K | Lys | L-lysine |
| H | His | L-histidine |
| Q | Gln | L-glutamine |
| E | Glu | L-glutamic acid |
| W | Trp | L-tryptophan |
| R | Arg | L-arginine |
| D | Asp | L-aspartic acid |
| N | Asn | L-asparagine |
| C | Cys | L-cysteine |

It should be noted that all amino acid residue sequences are represented herein by formulae whose left to right orientation is in the conventional direction of amino-terminus to carboxy-terminus. Furthermore, it should be noted that a dash at the beginning or end of an amino acid residue sequence indicates a bond to a further sequence of one or more amino acid residues up to a total of about fifty residues in the polypeptide chain.

Polypeptide and Peptide: Polypeptide and peptide are terms used interchangeably herein to designate a linear series of no more than about 50 amino acid residues connected one to the other by peptide bonds between the alpha-amino and carboxy groups of adjacent residues.

Protein: Protein is a term used herein to designate a linear series of greater than 50 amino acid residues connected one to the other as in a polypeptide.

B. Polypeptides

A polypeptide of the present invention consists essentially of 12 amino acid residues having a sequence represented by the formula
LGZWGCSGKHIC,
wherein Z is either isoleucine (I) or methionine (M).

The peptides of the invention contain at least two cysteine residues. Accordingly, the subject peptides can exist in various oxidative forms. In addition to the monomeric form in which the sulfhydryl group of the cysteine residues is reduced, there can also exist dimeric or polymeric forms in which sulfhydryl groups on

4

two or more peptide molecules become oxidized and form inter- and intrapeptide disulfide bonds. Because the polypeptides of the present invention possess two cysteine residues, they can form cyclic monomers or linear or cyclic dimers and linear polymers of various lengths. These various oxidative forms are considered part of the subject invention and are included in the terms "polypeptides" and "peptides".

A polypeptide of the present invention can be synthesized by any of the techniques that are known to those skilled in the polypeptide art, including recombinant DNA techniques. Synthetic chemistry techniques, such as a solid-phase Merrifield-type synthesis, are preferred for reasons of purity, antigenic specificity, freedom from undesired side products, ease of production and the like. An excellent summary of the many techniques available can be found in J.M. Steward and J.D. Young, "Solid Phase Peptide Synthesis", W.H. Freeman Co., San Francisco, 1969; M. Bodanszky, et al., "Peptide Synthesis", John Wiley & Sons, Second Edition, 1976 and J. Meienhofer, "Hormonal Proteins and Peptides", Vol. 2, p. 46, Academic Press (New York), 1983 for solid phase peptide synthesis, and E. Schroder and K. Kubke, "The Peptides", Vol. 1, Academic Press (New York), 1965 for classical solution synthesis. Appropriate protective groups usable in such synthesis are described in the above texts and in J.F.W. McOmie, "Protective Groups in Organic Chemistry", Plenum Press, New York, 1973.

In general, these methods comprise the sequential addition of one or more amino acid residues or suitably protected amino acid residues to a growing peptide chain. Normally, either the amino or carboxyl group of the first amino acid residue is protected by a suitable, selectively removable protecting group. A different, selectively removable protecting group is utilized for amino acids containing a reactive side group such as lysine.

Using a solid phase synthesis as exemplary, the protected or derivatized amino acid is attached to an inert solid support through its unprotected carboxyl or amino group. The protecting group of the amino or carboxyl group is then selectively removed and the next amino acid in the sequence having the complimentary (amino or carboxyl) group suitably protected is admixed and reacted under conditions suitable for forming the amide linkage with the residue already attached to the solid support. The protecting group of the amino or carboxyl group is then removed from this newly added amino acid residue, and the next amino acid (suitably protected) is then added, and so forth. After all the desired amino acids have been linked in the proper sequence, any remaining terminal and side group protecting groups (and solid support) are removed sequentially or concurrently, to afford the final polypeptide.

The polypeptides of this invention contain about 12 amino acid residues except where additional residues have been added at either terminus for the purpose of providing a "linker" by which the polypeptides of this invention can be conveniently affixed to a label or solid matrix, or carrier. Labels, solid matrices and carriers that can be used with the polypeptides of this invention are described hereinbelow.

Amino acid residue linkers are usually at least one residue and can be 40 or more residues, more often 1 to 10 residues and do not comprise sequences that mimic epitopes of AIDS-inducing viruses. Typical amino acid residues used for linking are tyrosine, cysteine, lysine, glutamic and aspartic acid, or the like. In addition, a polypeptide sequence of this invention can differ from the natural sequence by the sequence being modified by terminal-$NH_2$ acylation, e.g., acetylation, or thioglycolic acid amidation, by terminal-carboxlyamidation, e. g., with ammonia, methylamine, and the like.

When coupled to a carrier via a linker to form what is known in the art as a carrier-hapten conjugate, a polypeptide of the present invention is capable of inducing antibodies that immunoreact with and neutralize HIV-1.

C. Polypeptide Admixtures

The present invention also contemplates a composition comprising an admixture of at least two polypeptides wherein one of the polypeptides has an amino acid residue sequence represented by the formula:

LGZWGCSGKHIC

wherein Z is either isoleucine or methionine, (i.e., is chosen from the group of p3 and p4, whose respective amino acid residue sequences are shown in Table 1). A second of the polypeptides present in the admixture typically has an amino acid residue sequence that corresponds to a portion of a protein of a virus that causes or is associated with AIDS, such as HIV-1, HIV-2 and the like, and is capable of immunoreacting with antibodies induced by said protein. More preferably, the composition further contains, in admixture, at least one of the polypeptides p1, p2 and/or p5, whose amino acid residue sequence is also shown in Table 1. Typically, the various polypeptides present are present in about equimolar amount.

5

Table 1

| Designation | Amino Acid Residue Sequence | Virus |
|---|---|---|
| p1 | LGLWGCSGKLIC | HIV-1 |
| p2 | LGIWGCSGKLIC | HIV-1 |
| p3 | LGIWGCSGKHIC | HIV-1 |
| p4 | LGMWGCSGKHIC | HIV-1 |
| p5 | NSWGCAFRQVC | HIV-2 |

D. Inocula and Methods for Inducing HIV-1 Neutralizing Antibodies

The word "inoculum" in its various grammatical forms is used herein to describe a composition comprising a pharmaceutically acceptable aqueous diluent containing, as an active ingredient for the induction of HIV-1 neutralizing antibodies, a polypeptide having an amino acid residue sequence represented by the formula:

LGBWGCSGKJIC,

wherein B is either isoleucine (I), leucine (L) or methionine (M) and J is either histidine (H) or leucine (L). Preferred polypeptides having amino acid residue sequences represented by the above formula, are p1, p2, p3 and p4. Thus, when administered in an immunologically effective amount, an inoculum of this invention induces antibodies that immunoreact with and neutralize HIV-1.

When a polypeptide is used to induce antibodies it is to be understood that the polypeptide can be used alone, or linked to a carrier as a conjugate, or as a polypeptide polymer, but for ease of expression the various embodiments of the polypeptides of this invention are collectively referred to herein by the term "polypeptide", and its various grammatical forms.

For a polypeptide that contains fewer than about 35 amino acid residues, it is preferable to use the peptide bound to a carrier for the purpose of inducing the production of antibodies as already noted.

As also already noted, one or more additional amino acid residues can be added to the amino- or carboxy-termini of the polypeptide to assist in binding the polypeptide to a carrier. Cysteine residues added at the amino- or carboxy-termini of the polypeptide have been found to be particularly useful for forming conjugates via disulfide bonds. However, other methods well known in the art for preparing 30 conjugates can also be used. Exemplary additional linking procedures include the use of Michael addition reaction products, di-aldehydes such as glutaraldehyde, Klipstein et al., J. Infect. Dis., 147, 318-326 (1983) and the like, or the use of 35 carbodiimide technology as in the use of a water-soluble carbodiimide to form amide links to the carrier. For a review of protein conjugation or coupling through activated functional groups, see Aurameas, et al., Scand. J. Immunol., Vol. 8, Supp-1. 7, 7-23 (1978).

Useful carriers are well known in the art, and are generally proteins themselves. Exemplary of such carriers are keyhole limpet hemocyanin (KLH), edestin, thyroglobulin, albumins such as bovine serum albumin (BSA) or human serum albumin (HSA), red blood cells such as sheep erythrocytes (SRBC), tetanus toxoid, cholera toxoid, hepatitis B virus core particles or immunogenic fragments thereof as well as polyamino acids such as poly (D-lysine: D-glutamic acid), and the like.

The choice of carrier is more dependent upon the ultimate use of the inoculum and is based upon criteria not particularly involved in the present invention. For example, a carrier that does not generate an untoward reaction in the particular mammal to be inoculated should be selected.

The present inoculum contains an effective, immunogenic amount of a polypeptide of this invention, typically as a conjugate linked to a carrier. The effective amount of polypeptide per unit dose depends, among other things, on the species of mammal inoculated, the body weight of the mammal and the chosen inoculation regimen as is well known in the art. Inocula typically contain polypeptide concentrations of about 10 micrograms to about 500 milligrams per inoculation (dose), preferably about 50 micrograms to about 50 milligrams per dose.

The term "unit dose" as it pertains to the inocula of the present invention refers to physically discrete units suitable as unitary dosages for animals, each unit containing a predetermined quantity of active material calculated to produce the desired immunogenic effect in association with the required diluent; i.e., carrier, or vehicle. The specifications for the novel unit dose of an inoculum of this invention are dictated by and are directly dependent on (a) the unique characteristics of the active material and the particular immunologic effect to be achieved, and (b) the limitations inherent in the art of compounding such active material for immunologic use in animals and human subjects, as disclosed in detail herein, these being features of the present invention.

Inocula are typically prepared from the dried solid polypeptide-conjugate by dispersing the polypeptide-conjugate in a physiologically tolerable (acceptable) diluent such as water, saline or phosphate-buffered saline to form an aqueous composition.

Inocula can also include an adjuvant as part of the diluent. Adjuvants such as complete Freund's adjuvant (CFA), incomplete Freund's adjuvant (IFA) and alum are materials well known in the art, and are available commercially from several sources.

To induce HIV-1 virus neutralizing antibodies, an immunogenic amount of an inoculum of this invention is administered, typically by subcutaneous or intramuscular injection, to a mammal such as a mouse, rabbit, goat, horse, human and the like. The administered (inoculated) mammal is then maintained for a time period sufficient for the polypeptide present as active ingredient in the inoculum to induce neutralizing anti-HIV-1 antibodies. If desired, the antibodies so induced can then be harvested and used in preparations for passive immunization against HIV-1 or in diagnostic assays and systems to detect HIV-1-TMP in body samples.

E. Antibodies and Antibody Compositions

The term "antibody" in its various grammatical forms is used herein to refer to immunoglobulin molecules and immunologically active portions of immunoglobulin molecules, i.e., molecules that contain an antibody combining site or paratope. Exemplary antibody molecules are intact immunoglobulin molecules, substantially intact immunoglobulin molecules, and those portions of an immunoglobulin molecule that contain the paratope, including those portions known in the art as Fab, Fab′, F(ab′)$_2$ and F(v).

An antibody composition of the present invention is characterized as containing HIV-1 neutralizing antibody molecules that immunoreact with a polypeptide of the formula:

LGBWGCSGKJIC,

wherein B is either isoleucine, leucine or methionine and J is either histidine or leucine Preferably the polypeptide is p3 or p4. The antibody composition is further characterized as being substantially free of antibodies that immunoreact with HIV-1 TMP related polypeptides of the formula:

RILAVERYLKDQQLL, and

CTTAVPWNASWS.

An antibody composition of the present invention is typically produced by immunizing a mammal with an inoculum of the present invention and to thereby induce in the mammal antibody molecules having the appropriate polypeptide immunospecificity. The antibody molecules are then collected from the mammal and isolated to the extent desired by well known techniques such as, for example, by immunoaffinity chromatography. The antibody composition so produced can be used in, inter alia, the diagnostic methods and systems of the present invention to detect HIV-1 in a body sample.

The antibody compositions of this invention can be described as being oligoclonal as compared to naturally occurring polyclonal antibodies since they contain paratopes directed against relatively few epitopes as compared to the epitopes presented by an intact HIV-1 TMP molecule. Consequently, antibodies of this invention bind to the relatively few epitopes mimicked by the polypeptide, whereas naturally occurring antibodies raised to the whole TMP molecule bind to epitopes throughout the TMP molecule and are referred to as being polyclonal.

Monoclonal antibody compositions are also contemplated by the present invention. A monoclonal antibody composition contains, within detectable limits, only one species of antibody combining site capable of effectively binding HIV-1 TMP. Thus, a monoclonal antibody composition of the present invention typically displays a single binding affinity for HIV-1 TMP even though it may contain antibodies capable of binding proteins other than HIV-1 TMP.

Suitable antibodies in monoclonal form, typically whole antibodies, can be prepared using hybridoma technology described by Niman et al., Proc. Natl. Sci., U.S.A., 80:4949-4953 (1983). Briefly, to form the hybridoma from which the monoclonal antibody composition is produced, a myeloma or other self-perpetuating cell line is fused with lymphocytes obtained from the spleen of a mammal hyperimmunized with a polypeptide of this invention.

It is preferred that the myeloma cell line be from the same species as the lymphocytes. Typically, a mouse of the strain 129 GIX$^+$ is the preferred mammal. Suitable mouse myelomas for use in the present invention include the hypoxanthine-aminopterin-thymidine-sensitive (HAT) cell lines P3X63-Ag8.653, and Sp2/0-Ag14 that are available from the American Type Culture Collection, Rockville, MD, under the designations CRL 1580 and CAL 1581, respectively.

Splenocytes are typically fused with myeloma cells using polyethylene glycol (PEG) 1500. Fused hybrids are selected by their sensitivity to HAT. Hybridomas secreting the antibody molecules of this invention are identified using the enzyme linked immunosorbent assay (ELISA) described in Example 2.

A monoclonal antibody composition of the present invention can be produced by initiating a monoclonal hybridoma culture comprising a nutrient medium containing a hybridoma that secretes antibody molecules of the appropriate polypeptide specificity. The culture is maintained under conditions and for a time period sufficient for the hybridoma to secrete the antibody molecules into the medium. The antibody-containing medium is then collected. The antibody molecules can then be further isolated by well known techniques.

Media useful for the preparation of these compositions are both well known in the art and commercially available and include synthetic culture media, inbred mice and the like. An exemplary synthetic medium is Dulbecco's minimal essential medium (DMEM; Dulbecco et al., Virol. 8:396 (1959)) supplemented with 4.5 gm/1 glucose, 20 mm glutamine, and 20% fetal calf serum. An exemplary inbred mouse strain is the Balb/c.

The monoclonal antibody compositions produced by the above method can be used, for example, in diagnostic and therapeutic modalities wherein formation of an HIV-1 TMP-containing immunoreaction product is desired.

F. Diagnostic Systems

A diagnostic system in kit form of the present invention includes, in an amount sufficient for at least one assay, a polypeptide, polypeptide admixture, antibody composition or monoclonal antibody composition of the present invention, as a packaged reagent. Instructions for use of the packaged reagent are also typically included.

As used herein, the term "package" refers to a solid matrix or material such as glass, plastic, paper, foil and the like capable of holding within fixed limits a polypeptide, antibody composition or monoclonal antibody composition of the present invention. Thus, for example, a package can be a glass vial used to contain milligram quantities of a contemplated polypeptide or it can be a microtiter plate well to which microgram quantities of a contemplated polypeptide have been operatively affixed, i.e., linked so as to be capable of being immunologically bound by an antibody.

"Instructions for use" typically include a tangible expression describing the reagent concentration or at least one assay method parameter such as the relative amounts of reagent and sample to be admixed, maintenance time periods for reagent/sample admixtures, temperature, buffer conditions and the like.

In preferred embodiments, a diagnostic system of this invention contains polypeptide p3 and/or p4, preferably p3 and p4 in admixture.

Also preferred is a diagnostic system containing at least two polypeptides wherein a first of the polypeptides has an amino acid residue sequence represented by the formula:
LGZWGCSGKHIC,
wherein Z is either isoleucine or methionine. A second of the polypeptides is capable of immunoreacting with antibodies induced by a HIV, such as HIV-1, HIV-2 and the like, and preferably is chosen from the group of p1, p2 and p5. Each polypeptide can be separately packaged within the kit, e.g., present as part of separate solid supports. Preferably, the first and second polypeptides are present in admixture, preferably about equimolar admixture.

Further preferred is a diagnostic system having at least two solid supports, preferably separately packaged within the kit. A first solid support is comprised of a solid matrix having operatively affixed thereto an admixture of at least two polypeptides. A first of the polypeptides has an amino acid residue sequence represented by the formula:
LGZWGCSGKHIC,
wherein Z is either isoleucine or methionine, and a second of the polypeptides is chosen from the group of p1 and p2. A second solid support is comprised of a solid matrix having operatively affixed thereto polypeptide p5.

In preferred embodiments, a diagnostic system of the present invention further includes a label or indicating means capable of signaling the formation of a complex containing a polypeptide or antibody molecule of the present invention.

The word "complex" as used herein refers to the product of a specific binding reaction such as an antibody-antigen or receptor-ligand reaction. Exemplary complexes are immunoreaction products.

As used herein, the terms "label" and "indicating means" in their various grammatical forms refer to single atoms and molecules that are either directly or indirectly involved in the production of a detectable signal to indicate the presence of a complex. Any label or indicating means can be linked to or incorporated in an expressed protein, polypeptide, or antibody molecule that is part of an antibody or monoclonal antibody composition of the present invention, or used separately, and those atoms or molecules can be used alone or in conjunction with additional reagents. Such labels are themselves well-known in clinical diagnostic chemistry and constitute a part of this invention only insofar as they are utilized with otherwise novel proteins methods and/or systems.

The labeling means can be a fluorescent labeling agent that chemically binds to antibodies or antigens without denaturing them to form a fluorochrome (dye) that is a useful immunofluorescent tracer. Suitable fluorescent labeling agents are fluorochromes such as fluorescein isocyanate (FIC), fluorescein isothiocyante (FITC), 5-dimethylamine-1-naphthalenesulfonyl chloride (DANSC), tetramethylrhodamine isothiocyanate (TRITC), lissamine, rhodamine 8200 sulphonyl chloride (RB 200 SC) and the like. A description of immunofluorescence analysis techniques is found in DeLuca, "Immunofluorescence Analysis", in Antibody As a Tool, Marchalonis, et al., eds., John Wiley & Sons, Ltd., pp. 189-231 (1982).

In preferred embodiments, the indicating group is an enzyme, such as horseradish peroxidase (HRP), glucose oxidase, or the like. In such cases where the principal indicating group is an enzyme such as HRP or glucose oxidase, additional reagents are required to visualize the fact that a receptor-ligand complex (immunoreactant) has formed. Such additional reagents for HRP include hydrogen peroxide and an oxidation dye precursor such as diaminobenzidine. An additional reagent useful with glucose oxidase is 2,2′-azino-di-(3-ethyl-benzthiazoline-6-sulfonic acid) (ABTS).

Radioactive elements are also useful labeling agents and are used illustratively herein. An exemplary radiolabeling agent is a radioactive element that produces gamma ray emissions. Elements which themselves emit gamma rays, such as $^{124}$I, $^{125}$I, $^{128}$I, $^{132}$I and $^{51}$Cr represent one class of gamma ray emission-producing radioactive element indicating groups. Particularly preferred is $^{125}$I. Another group of useful labeling means are those elements such as $^{11}$C, $^{18}$F, $^{15}$O and $^{13}$N which themselves emit positrons. The positrons so emitted produce gamma rays upon encounters with electrons present in the animal's body. Also useful is a beta emitter, such $^{111}$indium of $^{3}$H.

The linking of labels, i.e., labeling of, polypeptides and proteins is well known in the art. For instance, antibody molecules produced by a hybridoma can be labeled by metabolic incorporation of radioisotope-containing amino acids provided as a component in the culture medium. See, for example, Galfre et al., Meth. Enzymol., 73:3-46 (1981). The techniques of protein conjugation or coupling through activated functional groups are particularly applicable. See, for example, Aurameas, et al., Scand. J. Immunol., Vol. 8 Suppl. 7:7-23 (1978), Rodwell et al., Biotech., 3:889-894 (1984), and U.S. Pat. No. 4,493,795.

The diagnostic systems can also include, preferably as a separate package, a specific binding agent. A "specific binding agent" is a molecular entity capable of selectively binding a reagent species of the present invention or a complex containing such a species, but is not itself a polypeptide or antibody molecule composition of the present invention. Exemplary specific binding agents are second antibody molecules, complement proteins or fragments thereof, S. aureus protein A, and the like. Preferably the specific binding agent binds the reagent species when that species is present as part of a complex.

In preferred embodiments, the specific binding agent is labeled. However, when the diagnostic system includes a specific binding agent that is not labeled, the agent is typically used as an amplifying means or reagent. In these embodiments, the labeled specific binding agent is capable of specifically binding the amplifying means when the amplifying means is bound to a reagent species-containing complex.

The diagnostic kits of the present invention can be used in an "ELISA" format to detect the presence or quantity of at least anti-HIV-1 antibodies in a body fluid sample such as serum, plasma or urine. "ELISA" refers to an enzyme-linked immunosorbent assay that employs an antibody or antigen bound to a solid phase and an enzyme-antigen or enzyme-antibody conjugate to detect and quantify the amount of an antigen or antibody present in a sample. A description of the ELISA technique is found in Chapter 22 of the 4th Edition of Basic and Clinical Immunology by D.P. Sites et al., published by Lange Medical Publications of Los Altos, CA in 1982 and in U.S. Patents No. 3,654,090; No. 3,850,752; and No. 4,016,043.

Thus, in preferred embodiments, a polypeptide, antibody molecule composition or monoclonal antibody molecule composition of the present invention can be affixed to a solid matrix to form a solid support that comprises a package in the subject diagnostic systems.

The reagent is typically affixed to the solid matrix by adsorption from an aqueous medium although other modes of affixation, well known to those skilled in the art, can be used.

Useful solid matrices are also well known in the art. Such materials are water insoluble and include the cross-linked dextran available under the trademark SEPHADEX from Pharmacia Fine Chemicals (Piscataway, NJ); agarose; beads of polystyrene beads about 1 micron to about 5 millimeters in diameter available from Abbott Laboratories of North Chicago, IL; polyvinyl chloride, polystyrene, cross-linked polyacrylamide, nitrocellulose- or nylon-based webs such as sheets, strips or paddles; or tubes, plates or the wells of a microtiter plate such as those made from polystyrene or polyvinylchloride.

The reagent species, labeled specific binding agent or amplifying reagent of any diagnostic system described herein can be provided in solution, as a liquid dispersion or as a substantially dry power, e.g., in lyophilized form. Where the indicating means is an enzyme, the enzyme's substrate can also be provided in a separate package of a system. A solid support such as the before-described microtiter plate and one or more buffers can also be included as separately packaged elements in this diagnostic assay system.

The packaging materials discussed herein in relation to diagnostic systems are those customarily utilized in diagnostic systems. Such materials include glass and plastic (e.g., polyethylene, polypropylene and polycarbonate) bottles, vials, plastic and plastic-foil laminated envelopes and the like.

## G. Assay Methods

The present invention contemplates a method for detecting the presence and preferably amount of antibodies against HIV-1 in a body fluid sample by producing a complex containing a polypeptide of the present invention and such antibodies. Those skilled in the art will understand that there are numerous well known clinical diagnostic chemistry procedures that can be utilized to form those complexes. Thus, while exemplary assay methods are described herein, the invention is not so limited.

Various heterogeneous and homogeneous assay protocols can be employed, either competitive or non-competitive, for detecting the presence, and preferably amount, of antibodies against HIV-1 TMP in a body fluid sample using a polypeptide of this invention. For example, the present invention contemplates a method for assaying a body sample for the presence of anti-HIV-1 antibodies comprising the steps of:

(a) Forming an immunoreaction admixture by admixing a body fluid sample with a polypeptide having an amino acid residue sequence represented by the formula:

LGZWGCSGKHIC,

wherein Z is either isoleucine or methionine. Preferably, the body fluid sample is provided as a known amount of blood or a blood derived product such as serum or plasma, although urine, saliva, semen, vaginal secretion or cerebral-spinal fluid (CSF) can also be used. Preferably the polypeptide is present as part of a solid support, e.g., a polypeptide of the present invention affixed to the inner walls of microtiter plate well, so that the immunoreaction admixture formed has a solid and a liquid phase.

(b) Maintaining the admixture under biological assay conditions for a predetermined time period such as about 10 minutes to about 16-20 hours at a temperature of about 4 degrees C to about 45 degrees C that is sufficient for any anti-HIV-1 antibodies present in the sample to immunoreact with (immunologically bind) the polypeptide to form a polypeptide-containing immunoreaction product.

Biological assay conditions are those that maintain the biological activity of the polypeptide molecules of this invention and the anti-HIV-1 antibodies sought to be assayed, and include a temperature range of about 4 degrees C to about 45 degrees C, a pH value range of about 5 to about 9 and an ionic strength varying from that of distilled water to that of about one molar sodium chloride. Methods for optimizing such conditions are well known in the art.

(c) Assaying for the presence of any polypeptide-containing immunoreaction product that formed, and thereby the presence of any anti-HIV-1 antibodies in the immunoreaction admixture is also determined. Preferably, the amount of any polypeptide-containing immunoreaction product formed is determined, and thereby the amount of anti-HIV-1 antibodies present in the sample.

Assaying for the presence of any polypeptide-containing (anti-HIV-1 antibody-containing) immunoreaction product, either directly or indirectly, can be accomplished by assay techniques well known in the art. For instance, in preferred embodiments, the polypeptide-containing immunoreaction product of step (b) is further prepared for assaying according to step (c) by the following steps:

(i) Admixing a biologically active labeled specific binding agent with the polypeptide-containing immunoreaction product to form a labeling-reaction admixture. The labeled specific binding agent is capable of binding to any immunoglobulin present in the polypeptide-containing immunoreaction product to form a labeled complex. Preferably, the labeled specific binding agent is comprised of second antibody molecules such as xenogeneic anti-human Fc antibodies. More preferably, the labeled specific binding reagent is immunoglobulin class specific.

(ii) The labeling-reaction admixture so formed is maintained under biological assay conditions for a predetermined time period sufficient for the labeled specific binding agent to bind to any anti-HIV-1 antibodies present as polypeptide-containing immunoreaction product to form a labeled complex.

Assaying for the presence of the labeled complex provides an assay for the presence of anti-HIV-1 antibodies in the sample. In preferred embodiments, the amount of the labeled specific binding agent bound as part of the complex is determined, and thereby the presence and amount of anti-HIV-1 antibodies in the sample can be determined. That amount can be zero, thereby indicating no anti-HIV-1 antibodies are present in the sample, within the limits that can be detected. Methods for assaying for the presence and amount of a labeled specific binding agent depend on the label used, such labels and assay methods being

10

well known in the art.

Alternatively, homogeneous assay methods such as those described in U.S. Patents No. 4,536,479; No. 4,233,401; No. 4,233,402 and No. 3,996,345, can be used to detect the polypeptide-containing immunoreaction product of step (c).

## Examples

### 1. Polypeptide Synthesis

Polypeptides p1, p2, p3, p4 and p5 were synthesized using the classical solid-phase technique described by Merrifield, Adv. Enzymol., 32:221-96 (1969) as adapted for use with a Model 430A automated peptide synthesizer (Applied Biosystems, Foster City, CA). Polypeptide resins were cleaved by hydrogen fluoride, extracted and analyzed for purity by high-performance liquid chromatograph using a reverse-phase C18 column. (Waters Associates, Milford, MA).

The amino acid residue sequence of polypeptide p5, which is derived from the sequence of an HIV-2 isolate, is shown in Table 1 of Section C. For ease of comparison, the amino acid residue sequences of polypeptides p1, p2, p3 and p4, which are derived from 4 different HIV-1 isolates, are shown in Table 2.

## Table 2

| Peptide Designation | Amino Acid Residue Sequence[a] | | | | | | | | | | | |
|---|---|---|---|---|---|---|---|---|---|---|---|---|
| | 1 | 2 | 3 | 4 | 5 | 6 | 7 | 8 | 9 | 10 | 11 | 12 |
| p2 | L | G | I | W | G | C | S | G | K | L | I | C |
| p1 | –[b] | – | L | – | – | – | – | – | – | – | – | – |
| p3 | – | – | – | – | – | – | – | – | – | H | – | – |
| p4 | – | – | M | – | – | – | – | – | – | H | – | – |

[a] The amino acid residue sequence of each polypeptide corresponds to a portion of a known HIV-1 gp41 amino acid residue sequence.

[b] A dash indicates the amino acid residue is the same as that found in the same position as in the polypeptide p2 sequence, whereas the presence of an amino acid that is different from that found in the p2 sequence is designated with the letter corresponding to the different or "substituted" residue. Thus, p1 has an amino acid residue sequence that is identical to that of p2 except for a leucine for isoleucine substitution at residue position number 3.

2. ELISA System and Procedure

One hundred microliters (ul) of phosphate buffered saline (PBS) containing 1 microgram (ug) of polypeptide p1, p2, p3, p4 or p5 were admixed into the wells of polyvinyl, 96-well microliter plates (Microtest III, Falcon). The plates were then maintained for about 16 hours at 37 degrees C to permit the PBS to evaporate and the polypeptides to adsorb onto (coat by operatively linking thereto) the walls of the wells. One hundred fifty ul of ELISA diluent [PBS containing 0.2% polyoxyethylene (20) sorbitan monolaurate (Tween®20), 10% heat-inactivated fetal calf serum (FCS) and 0.5 millimolar (mM) thimerosal] were then admixed into each well to block excess protein binding sites.

After maintaining the diluent-containing wells for 1 hour at about 23 degrees C, the diluent was removed by shaking or aspiration, thus forming a diagnostic system of the present invention, i.e., a microtiter plate well having a polypeptide of the present invention operatively affixed to its inner walls (a polypeptide-coated well).

One hundred ul of each serum diluted 1:64 in ELISA diluent were admixed into a polypeptide-coated well. The resulting solid-liquid phase immunoreaction admixture was maintained at about 23 degrees C for 90 minutes to permit formation of polypeptide-containing immunoreaction products. The diluted sera were then removed from the wells by aspiration using an ELISA washer (Immuno Wash® 12, Nunc). Each well was then washed 4 times with ELISA wash (PBS containing 0.2% Tween20) and aspirated dry.

One hundred ul of a horseradish peroxidase-labeled goat anti-human IgG, affinity-purified, (Boehringer Mannheim Biochemicals) diluted 1:30,000 in PBS containing 10% heat-inactivated FCS and 0.5 mM thimersol, were then admixed into each well. The resulting labeling-reaction admixture was maintained for 90 minutes at about 23 degrees C to permit formation of polypeptide-containing labeled complexes. The unreacted labeled-antibody was removed by shaking and each well was washed and aspirated dry as previously described.

One hundred ul of chromogenic substrate solution [citrate buffer (500 ml deionized water containing 5.0 g anhydrous citric acid and 7.0 g $Na_2HPO_4$, pH 5) containing 0.4 mg/ml 0-phenylenediamine (OPD) and 0.01% $H_2O_2$] were admixed into each well to form a developing-reaction admixture. After maintaining the developing-reaction admixture for 30 minutes at about 23 degrees C, 100 ul of 1 M HCl were admixed into each well to stop the developing-reaction. The resulting solutions were assayed for absorbance at 492 nanometers ($OD_{492}$) using an ELISA reader (Titertek Multiscan®, Flow Laboratories, Inglewood, CA). Seropositivity (HIV infection) was defined as any $OD_{492}$ value greater than the mean $OD_{492}$ value plus 3 standard deviations of 24 normal control (noninfected) sera. All sera were assayed at least 3 times.

Sera from HIV-1 infected United States patients were collected in San Diego, CA (n = 84) and by the Centers for Disease Control (CDC) locations throughout the United States (n = 79). The panel of 40 HIV-1 positive sera included specimens from 12 asymptomatic seropositive patients (CDC Group II), 13 patients with generalized lymphodemopathy (CDC Group III) or AIDS-related complex ARC; CDC Group IV-A), and 15 patients with overt AIDS (CDC Group IV-C or IV-D). See Centers for Disease Control, Morbid. Mortal Weekly Rep., 35:334 (1976).

The sera from Zairian HIV-1 infected patients and healthy, normal Zairian controls were collected in Kinshasa in 1983 and were characterized by Brun-Vezinet et al., Science, 226:453 (1984).

The HIV-2 positive sera were collected in Guinea Bissau in 1980 and characterized by immunoblotting and immunofluorescence against HIV-1, HIV-2 and STLV antigens. Fultz et al., Third International Conference on AIDS, abstract MP.72 (1987).

All serum samples were coded, divided into aliquots and stored at -20 degrees C.

The results of assaying the above described sera for the presence of anti-HIV antibodies using polypeptides p1, p2, p3, p4 and p5 are shown in Table 3.

From Table 3, it can be seen that polypeptide p2, whose amino acid residue sequence is homologous to the carboxy-terminal 12 residues of peptide 39 described in the Cosand patent, immunoreacted with 100% of the HIV-1-infected sera obtained from United States patients, but only 88.2% of Zairian HIV-1-infected sera were detected.

Table 3

| Characterization of Sera From Zairian AIDS Patients | | | | | | | | | |
|---|---|---|---|---|---|---|---|---|---|
| Serum | HIV Culture | Western Blot | Whole Virus | ELISA Antigens | | | | | |
| | | | | p1 | p2 | p3 | p4 | p5 | |
| Z-9 | + | p24+,p41+/- | + | -[a] | + | + | + | - | |
| Z-23 | + | p24+,p41+/- | + | - | - | + | + | - | |
| Z-24 | + | p25+ | + | + | + | + | + | - | |
| Z-29 | + | ND[b] | + | + | + | - | - | - | |
| Z-31 | + | p24+,p41+ | + | + | + | + | + | - | |
| Z-34 | + | p24+,p41- | + | - | - | + | + | - | |
| Z-47 | + | P24+,p41- | + | - | - | + | + | - | |
| Z-50 | - | p24+,p41+ | + | - | - | + | + | ND | |
| Z-51 | - | p41+ | + | + | + | + | + | - | |
| Z-52 | + | p25+,p41+ p60+ | + | + | + | + | + | - | |
| Z-68 | + | ND | + | + | + | - | + | - | |
| Z-72 | + | p25+ | + | + | + | + | + | - | |
| Z-75 | + | p18+,p25+ p41+,p60+ | + | + | + | + | + | - | |
| Z-81 | + | p18+,p25+ p41+,P60+ | + | + | + | + | + | - | |

[a] + = positive; - = negative (The cutoff for positivity for the peptide ELISA was defined as the mean $OD_{492}$ plus 3 standard deviations for a panel of 24 negative control sera. Specimens were scored as positive or negative at a serum dilution of 1:64.)
[b] ND = not done, insufficient quantity of specimen.

Similarly, polypeptide p1, whose amino acid residue sequence contains a leucine for isoleucine substitution, relative to polypeptide p2, immunoreacted with 99.4% of the HIV-1-infected United States sera but only 86.8% of the HIV-1-infected Zairian sera.

In contrast to polypeptides p1 and p2, the results shown in Table 3 indicate that the polypeptides of the present invention, i.e., polypeptides p3 and p4, detected anti-HIV-1 TMP antibodies in 94.1% and 97.1%, respectively, of the Zairian AIDS sera tested. The difference in degree of sensitivity between the polypeptides of this invention and polypeptides p1 and p2 was unexpected because 1) polypeptides p1 and p2 appear to have equivalent sensitivities for HIV-1-TMP antibodies in both United States and Zairian sera, and 2) the amino acid residue sequences of polypeptides p1, p3 and p4 and are all derived from Zairian HIV-1 isolates but they have unexpected differences in sensitivity for anti-HIV-1-TMP antibodies.

The reason(s) for the above described differences in sensitivity are unknown. However, those differences suggest that polypeptides p3 and/or p4, can be used with polypeptides such as p1 and p2 to achieve a greater level of sensitivity for exposure to HIV-1. In addition, the differences in sensitivity shown in Table 3 indicate that a polypeptide of this invention can be used in combination with an HIV-2 specific polypeptide such as p5 to achieve a higher level of sensitivity for exposure to HIV-1 or HIV-2 than can be achieved through use of a HIV-2 derived polypeptide alone.

Five of the Zairian AIDS sera that did not react with polypeptide p1, 1 that did not react with either polypeptide p3 or p4, and 8 that reacted with p1 and p4 were assayed for the presence of infectious HIV-1 according to the culture method of McCormick et al., Am. J. Trop. Med. Hyg., 36:102 (1987). In addition, those sera were assayed for the presence of anti-HIV-1 antibodies using a commercial ELISA diagnostic system that uses whole-virus (viral lysate) antigens (Litton Bionetics, Kensington, MD) and for antibodies to specific HIV-1 proteins by Western immunoblotting using CDC HIV-1 strain 451 as antigen source. The results of those assays, shown in Table 4, indicate that polypeptides p3 and p4 reacted with all 5 Zairian sera that were non-reactive with polypeptide p1. In addition, because all 5 of the polypeptide p1 negative sera were found to contain infectious HIV-1 but were negative or equivocal by Western blot assay, the diagnostic systems of the present invention represent immunoassay systems having improved sensitivity for anti HIV-1 TMP antibodies.

### 3. Antibody Production and Neutralization Assay

Polypeptide p1 was coupled to keyhole limpet hemocyanin (KLH) carrier protein through the carboxy-terminal cysteine residue using the cross-linker m-maleimidobenzoyl-N-hydroxysuccinimide ester according to the method of Lin et al., Biochem. 18:690-697 (1979). After preimmunization sera samples were obtained, two rabbits were each injected on day 0 at six subcutaneous locations with 250 ug of coupled polypeptide emulsified in 1 ml of complete Freund's adjuvant. On day 14, the rabbits were injected subcutaneously with 250 ug coupled polypeptide in incomplete Freund's adjuvant. On day 21, they were injected intraperitoneally with 250 ug coupled polypeptide in 1 ml of an alum suspension. Rabbit sera were collected at 7 and 14 days after the third injection and were stored at -20 degrees C. Antibody levels were determined by the ELISA procedure of Example 2.

The ability of the rabbit anti-polypeptide p1 antisera to neutralize HIV-1 infectivity was determined by a reverse transcriptase (RT) activity assay similar to that reported by Chanh et al., EMBO, 5:3065-71 (1986). The results of this study, shown in Table 4, indicate that immunization with an inoculum of this invention induces HIV-1 neutralizing antibodies.

## Table 4

| Rabbit Anti-polypeptide Serum Number | $CPM^a X10^3$ | % Inhibition[b] of RT Activity |
|---|---|---|
| V3343 | 12,246 | 95 |
| V3344 | 55,202 | 77 |
| Control[c] | 218,210 | |
| Control | 264,122 | |

[a]   CPM = counts per minute of $[^3H]$TTP.

[b]   % Inhibition of RT Activity (% Neutralization) =

$$1 - [\frac{CPM\ RT\ assay\ of\ anti\text{-}polypeptide\ antiserum}{Average\ CPM\ RT\ assay\ of\ control\ sera}]\ X\ 100\%$$

[c]   Control = preimmune rabbit serum.

The foregoing specification, including the specific embodiments and examples, is intended to be illustrative of the present invention and is not to be taken as limiting. Numerous other variations and modifications can be effected without departing from the true spirit and scope of the present invention.

**Claims**

1.  A polypeptide consisting essentially of 12 amino acid residues having an amino acid residue sequence represented by the formula:
    LGZWGCSGKHIC,
    wherein Z is either isoleucine or methionine.

2.  A composition comprising an admixture of at least two polypeptides wherein a first of said polypeptides consists essentially of 12 amino acid residues having a sequence represented by the formula:
    LGZWGCSGKHIC,
    wherein Z is either isoleucine or methionine, and a second of said polypeptides is capable of immunoreacting with antibodies induced by a human immunodeficiency virus.

3. The composition according to claim 2 wherein a second of said polypeptides has an amino acid residue sequence represented by a formula selected from the group consisting of:
LGLWGCSGKLIC,
LGIWGCSGKLIC, and
NSWGCAFRQVC.

4. A method of assaying for the presence of anti-HIV-1 antibodies in a body fluid sample comprising the steps of:
a) forming an immunoreaction admixture by admixing a body fluid sample with a polypeptide consisting essentially of 12 amino acid residues having a sequence represented by a formula:
LGZWGCSGKHIC,
wherein Z is either isoleucine or methionine;
b) maintaining said immunoreaction admixture under biological assay conditions for a time period sufficient for any anti-HIV-1 antibodies present in the sample to immunoreact with said polypeptide and form a polypeptide-containing immunoreaction product; and
c) assaying for the presence of any polypeptide-containing immunoreaction product that formed, and thereby the presence of any anti-HIV-1 antibodies in said sample.

5. The method according to claim 4 wherein said polypeptide-containing immunoreaction product is further prepared for assaying according to step (c) by:
(i) forming a labeling reaction admixture by admixing with said polypeptide-containing immunoreaction product labeled antibody molecules capable of binding to any human immunoglobulin present in said immunoreaction product, and
(ii) maintaining said labeling reaction admixture so formed under biological assay conditions for a time period sufficient for said labeled antibodies to immunoreact with any anti-HIV-1 antibodies present as polypeptide-containing immunoreaction product to form a labeled complex.

6. The method according to claim 5 wherein prior to said admixture of step (a) said polypeptide is affixed to a solid matrix.

7. A method of assaying for the presence of anti-HIV antibodies in a body fluid sample comprising the steps of:
(a) forming an immunoreaction admixture by admixing a body fluid sample with a composition comprising an admixture of at least two polypeptides wherein said first of said polypeptides consists essentially of 12 amino acid residues and has a sequence represented by the formula:
LGZWGCSGKHIC,
wherein Z is either isoleucine or methionine, and a second of said polypeptides is represented by a formula selected from the group consisting of:
LGLWGCSGKLIC,
LGIWGCSGKLIC, and
NSWGCAFRQVC;
b) maintaining said immunoreaction admixture under biological assay conditions for a time period sufficient for any anti-HIV antibodies present in the sample to immunoreact with said polypeptide and form a polypeptide-containing immunoreaction product; and
c) assaying for the presence of any polypeptide-containing immunoreaction product that formed, and thereby the presence of any anti-HIV antibodies in said sample.

8. The method according to claim 7 wherein said polypeptide-containing immunoreaction product is further prepared for assaying according to step (c) by:
(i) forming a labeling reaction admixture by admixing with said polypeptide-containing immunoreaction product labeled antibody molecules capable of binding to any human immunoglobulin present as immunoreaction product, and
(ii) maintaining said labeling reaction admixture so formed under biological assay conditions for a time period sufficient for said labeled antibodies to immunoreact with any anti-HIV antibodies present as polypeptide-containing immunoreaction product to form a labeled complex.

9. The method according to claim 7 wherein prior to said admixture of step (a) said polypeptide is affixed to a solid matrix.

15

**10.** A diagnostic system in kit form for assaying for the presence of anti-HIV-1 antibodies in a body fluid sample comprising a package containing a polypeptide having an amino acid residue sequence represented by the formula:
LGZWGCSGKHIC,
wherein Z is either isoleucine or methionine.

**11.** A diagnostic system in kit form for assaying for the presence of anti-HIV antibodies in a body fluid sample comprising a package containing at least two polypeptides wherein a first of said polypeptides has an amino acid residue sequence represented by the formula:
LGZWGCSGKHIC,
wherein Z is either isoleucine or methionine, and a second of said polypeptides is capable of immunoreacting with antibodies induced by a human immunodeficiency virus.

**12.** The diagnostic system according to claim 11 wherein said second of said polypeptides has an amino acid residue sequence represented by a formula selected from the group consisting of:
LGLWGCSGKLIC,
LGIWGCSGKLIC, and
NSWGCAFRQVC.

**13.** The diagnostic system of claim 11 wherein said first and second polypeptides are admixed.

**14.** The diagnostic system according to claim 11 further including, in a separate package, a labeled specific binding agent for signaling the presence of a polypeptide-containing immunoreaction product.

**15.** The diagnostic system according to claim 11 wherein said polypeptide is affixed to a solid matrix.

**16.** A diagnostic system in kit form comprising:
(a) a first solid support comprised of a solid matrix having operatively affixed thereto a polypeptide having an amino acid residue sequence represented by the formula:
LGZWGCSGKHIC,
wherein Z is either isoleucine or methionine; and
(b) a second solid support comprised of a solid matrix having operatively affixed thereto a polypeptide having an amino acid residue sequence represented by a formula selected from the group consisting of:
LGLWGCSGKLIC,
LGIWGCSGKLIC, and
NSWGCAFRQVC.

**17.** A diagnostic system in kit form comprising:
(a) a first solid support comprised of a solid matrix having operatively affixed thereto an admixture of at least two polypeptides wherein a first of said polypeptides has an amino acid residue sequence represented by the formula:
LGZWGCSGKHIC,
wherein Z is either isoleucine or methionine, and a second of said polypeptides has an amino acid residue sequence represented by a formula selected from the group consisting of:
LGLWGCSGKLIC, and
LGIWGCSGKLIC; and
(b) a second solid support comprised of a solid matrix having operatively affixed thereto a polypeptide having an amino acid residue sequence represented by the formula:
NSWGCAFRQVC.

**Patentansprüche**

**1.** Polypeptid, bestehend im wesentlichen aus 12 Aminosäureresten mit einer Aminosäuresequenz, die durch die Formel:
LGZWGCSGKHIC
dargestellt wird, wobei Z entweder Isoleucin oder Methionin ist.

2. Zusammensetzung, umfassend eine Mischung von mindestens zwei Polypeptiden, worin ein erstes dieser Polypeptide im wesentlichen aus 12 Aminosäureresten besteht, deren Sequenz durch die Formel:

LGZWGCSGKHIC

dargestellt wird, wobei Z entweder Isoleucin oder Methionin ist, und ein zweites dieser Polypeptide mit durch ein Human-Immundefizienz-Virus induzierten Antikörpern immunreagieren kann.

3. Zusammensetzung nach Anspruch 2, wobei ein zweites dieser Polypeptide eine Aminosäuresequenz hat, die durch eine Formel dargestellt wird, die aus der aus:

LGLWGCSGKLIC,

LGIWGCSGKLIC, und

NSWGCAFRQVC

bestehenden Gruppe ausgewählt ist.

4. Verfahren zum Testen auf die Gegenwart von anti-HIV-1-Antikörpern in einer Körperflüssigkeitsprobe, umfassend die folgenden Schritte:

a) das Bilden einer Immunreaktionsmischung durch Mischen einer Körperflüssigkeitsprobe mit einem Polypeptid, das im wesentlichen aus 12 Aminosäureresten mit der durch die Formel:

LGZWGCSGKHIC

dargestellten Sequenz besteht, worin Z entweder Isoleucin oder Methionin ist;

b) das Aufbewahren der Immunreaktionsmischung unter biologischen Testbedingungen für einen Zeitraum, der für jeden in der Probe vorhandenen anti-HIV-Antikörper ausreichend ist, eine Immunreaktion mit dem Polypeptid einzugehen und ein Polypeptid-haltiges Immunreaktionsprodukt zu bilden; und

c) das Testen auf die Gegenwart eines Polypeptid-haltigen Immunreaktionsproduktes, das sich gebildet hat, und dadurch auf die Gegenwart jedes anti-HIV-1-Antikörpers in der Probe.

5. Verfahren nach Anspruch 4, worin das Polypeptid-haltige Immunreaktionsprodukt weiter für den Test gemäß Schritt (c) vorbereitet wird durch:

i) Bilden einer Markierungsreaktionsmischung durch Mischen von markierten Antikörpermolekülen, die an jedes Human-Immunglobulin, das in dem Immunreaktionsprodukt vorhanden ist, binden können, mit dem Polypeptid-haltigen Immunreaktionsprodukt, und

ii) Aufbewahren der so gebildeten Markierungsreaktionsmischung unter biologischen Testbedingungen für einen Zeitraum, der für die markierten Antikörper ausreichend ist, mit jedem als Polypeptid-haltiges Immunreaktionsprodukt vorhandenen anti-HIV-1-Antikörper unter Bildung eines markierten Komplexes eine Immunreaktion einzugehen.

6. Verfahren nach Anspruch 5, wobei das Polypeptid vor dem Mischen gemäß Schritt (a) an eine feste Matrix angeheftet wird.

7. Verfahren zum Testen auf die Gegenwart von anti-HIV-Antikörpern in einer Körperflüssigkeitsprobe, umfassend die folgenden Schritte:

a) das Bilden einer Immunreaktionsmischung durch Mischen einer Körperflüssigkeitsprobe mit einer Zusammensetzung, die eine Mischung von mindestens zwei Polypeptiden umfaßt, wobei das erste der Polypeptide im wesentlichen aus 12 Aminosäureresten besteht und eine durch die Formel:

LGZWGCSGKHIC

dargestellte Sequenz hat, wobei Z entweder Isoleucin oder Methionin ist, und ein zweites der Polypeptide durch eine Formel dargestellt wird, die aus der aus:

LGLWGCSGKLIC,

LGIWGCSGKLIC, und

NSWGCAFRQVC

bestehenden Gruppe ausgewählt ist,

b) das Aufbewahren der Immunreaktionsmischung unter biologischen Testbedingungen für einen Zeitraum, dar für jeden in der Probe vorhandenen anti-HIV-Antikörper ausreichend ist, mit dem Polypeptid eine Immunreaktion einzugehen und ein Polypeptid-haltiges Immunreaktionsprodukt zu bilden, und

c) das Testen auf die Gegenwart eines Polypeptid-haltigen Immunreaktionsproduktes, das sich gebildet hat, und dadurch auf die Gegenwart jedes anti-HIV-Antikörpers in der Probe.

**8.** Verfahren nach Anspruch 7, wobei das Polypeptid-haltige Immunreaktionsprodukt weiter zum Test gemäß Schritt (c) vorbereitet wird durch:

i) Bilden einer Markierungsreaktionsmischung durch Mischen von markierten Antikörpermolekülen, die an jedes Human-Immunglobulin, das als Immunreaktionsprodukt vorhanden ist, binden können,

ii) Aufbewahren der so gebildeten Markierungsreaktionsmischung unter biologischen Testbedingungen für einen Zeitraum, der für die markierten Antikörper ausreichend ist, mit jedem als Polypeptid-haltiges Immunreaktionsprodukt vorhandenen anti-HIV-Antikörper unter Bildung eines markierten Komplexes eine Immunreaktion einzugehen.

**9.** Verfahren nach Anspruch 7, wobei das Polypeptid vor dem Mischen von Schritt (a) an eine feste Matrix fixiert wird.

**10.** Diagnostisches System in Kitform zum Testen auf die Gegenwart von anti-HIV-1-Antikörpern in einer Körperflüssigkeitsprobe, umfassend eine Packung, die ein Polypeptid mit einer durch die folgende Formel dargestellten Aminosäuresequenz enthält:
LGZWGCSGKHIC
wobei Z entweder Isoleucin oder Methionin ist.

**11.** Diagnostisches System in Kitform zum Testen auf die Gegenwart von anti-HIV-Antikörpern in einer Körperflüssigkeitsprobe, umfassend eine Packung, die mindestens zwei Polypeptide enthält, wobei ein erstes dieser Polypeptide eine durch die Formel:
LGZWGCSGKHIC
dargestellte Aminosäuresequenz hat, wobei Z entweder Isoleucin oder Methionin ist, und ein zweites der Polypeptide mit durch ein Human-Immundefizienz-Virus induzierten Antikörpern eine Immunreaktion eingehen kann.

**12.** Diagnostisches System nach Anspruch 11, wobei das zweite der Polypeptide eine Aminosäuresequenz hat, die durch eine Formel dargestellt wird, die aus der aus:
LGLWGCSGKLIC,
LGIWGCSGKLIC, und
NSWGCAFRQVC
bestehenden Gruppe ausgewählt ist.

**13.** Diagnostisches System nach Anspruch 11, wobei das erste und zweite Polypeptid gemischt werden.

**14.** Diagnostisches System nach Anspruch 11, das weiter in einer getrennten Packung ein markiertes spezifisches Bindemittel für das Anzeigen der Gegenwart eines Polypeptid-haltigen Immunreaktionsproduktes enthält.

**15.** Diagnostisches System nach Anspruch 11, wobei das Polypeptid an eine feste Matrix fixiert ist.

**16.** Diagnostisches System in Kitform, umfassend:

a) einen ersten festen Träger, der eine feste Matrix mit funktionell daran gebundenem Polypeptid mit einer durch die Formel:
LGZWGCSGKHIC,
dargestellten Aminosäuresequenz umfaßt, wobei Z entweder Isoleucin oder Methionin ist, und
b) einen zweiten festen Träger, der eine feste Matrix mit funktionell daran gebundenem Polypeptid mit einer Aminosäuresequenz umfaßt, die durch eine Formel dargestellt wird, die aus der aus:
LGLWGCSGKLIC,
LGIWGCSGKLIC, und
NSWGCAFRQVC
bestehenden Gruppe ausgewählt ist.

**17.** Diagnostisches System in Kitform, umfassend:

a) einen ersten festen Träger, der eine feste Matrix mit einer funktionell daran gebundenen Mischung von mindestens zwei Polypeptiden umfaßt, wobei ein erstes dieser Polypeptide eine durch die Formel:
LGZWGCSGKHIC

18

dargestellte Aminosäuresequenz hat,
wobei Z entweder Isoleucin oder Methionin ist, und ein zweites dieser Polypeptide eine Aminosäuresequenz hat, die durch eine Formel dargestellt wird, die aus der aus:
LGLWGCSGKLIC, und
LGIWGCSGKLIC
bestehenden Gruppe ausgewählt ist, und
b) einen zweiten festen Träger, der eine feste Matrix mit einem funktionell daran gebundenem Polypeptid mit einer durch die Formel:
NSWGCAFRQVC
dargestellten Aminosäuresequenz enthält.

## Revendications

1. Polypeptide consistant essentiellement de résidus de 12 amino acide ayant une séquence résidu amino-acide représentée par la formule :
LGZWGCSGKHIC,
   dans laquelle Z est soit l'isoleucine soit la méthionine.

2. Composition comprenant un ajout en mélange d'au moins deux polypeptides dans laquelle un premier desdits polypeptides consiste essentiellement de résidus de 12 amino-acide ayant une séquence représentée par la formule :
LGZWGCSGKHIC,
   dans laquelle Z est soit l'isoleucine soit la méthionine, et un second desdits polypeptides est capable d'immunoréaction avec des anticorps induits par un virus d'immunodéficience humaine.

3. Composition selon la revendication 2 dans laquelle un second desdits polypeptides a une séquence résidu de amino-acide représentée par une formule sélectionnée dans le groupe consistant en :
LGLWGCSGKLIC,
LGIWGCSGKLIC, et
NSWGCAFRQVC.

4. Méthode d'analyse de la présence d'anticorps anti-HIV-1 dans un échantillon de fluide corporel comprenant les étapes de :
   a) formation d'un ajout en mélange d'immunoréaction en ajoutant et mélangeant un échantillon de fluide corporel avec un polypeptide consistant essentiellement de résidus de 12 amino-acide ayant une séquence représentée par la formule :
   LGZWGCSKHIC,
      dans laquelle Z est soit l'isoleucine soit la méthionine;
   b) maintien dudit ajout en mélange d' immunoréaction dans des conditions d'analyse biologique pendant une période de temps suffisante pour que des anticorps anti-HIV-1 présents dans l'échantillon réagissent immunitairement avec ledit polypeptide et forment un produit d'immunoréaction contenant un polypeptide ; et
   c) analyse de la présence d'un produit d'immunoréaction contenant un polypeptide formé, et par là la présence d'un anticorps anti-HIV-1 dans ledit échantillon.

5. Méthode selon la revendication 4 dans laquelle ledit produit d'immunoréaction contenant un polypeptide est de plus préparé pour analyser selon l'étape (c) par :
   (i) formation d'un ajout en mélange de réaction de marquage en ajoutant et en mélangeant avec ledit polypeptide contenant des molécules anticorps marquées de produit d'immunoréaction capables de s'agglomérer à une immunoglobuline humaine présente dans ledit produit d'immunoréaction, et
   (ii) maintien dudit ajout en mélange de réaction de marquage de façon à former sous des conditions d'analyse biologique pendant une période de temps suffisante pour que lesdits anticorps marqués réagissent avec des anticorps anti-HIV-1 présents comme produit d'immunoréaction contenant un polypeptide pour former un complexe marqué.

6. Méthode selon la revendication 5 dans laquelle avant ledit ajout en mélange de l'étape (a) ledit polypeptide est fixé sur une matrice solide.

EP 0 329 761 B1

**7.** Méthode d'analyse de la présence d'anticorps anti-HIV-1 dans un échantillon de fluide corporel comprenant les étapes de :

(a) formation d'un ajout en mélange d'immunoréaction en ajoutant en mélange un échantillon de fluide corporel à une composition comprenant un ajout en mélange d'au moins deux polypeptides dans laquelle ledit premier desdits polypeptides consiste essentiellement en résidus de 12 amino-acide et a une séquence représentée par la formule :
LGZWGCSGKHIC,
dans laquelle Z est soit l'isoleucine soit la méthionine, et un second desdits polypeptides est représenté par une formule sélectionnée dans le groupe consistant en :
LGLWGCSGKLIC,
LGIWGCSGKLIC, et
NSWGCAFRQVC;

b) maintien dudit ajout en mélange d'immunoréaction sous des conditions d'analyse biologique pendant une période de temps suffisante pour que des anticorps anti-HIV présents dans l'échantillon réagissent avec ledit polypeptide et forment un produit d'immunoréaction contenant un polypeptide et

c) analyse de la présence d'un produit d'immunoréaction contenant un polypeptide qui est formé, et par là la présence d'anticorps anti-HIV dans ledit échantillon.

**8.** Méthode selon la revendication 7 dans laquelle ledit produit d'immunoréaction contenant un polypeptide est de plus préparé pour analyse selon l'étape (c) par :

(i) formation d'un ajout en mélange de réaction de marquage par ajout en mélange avec lesdites molécules d'anticorps marquées du produit d'immunoréaction contenant un polypeptide capable de s'agglomérer à une immunoglobuline humaine présente comme produit d'immunoréaction, et

(ii) maintien dudit ajout en mélange de réaction de marquage pour former sous des conditions d'analyse biologique pendant une période de temps suffisante pour que lesdits anticorps marqués réagissent avec les anticorps anti-HIV présents comme produit d'immunoréaction contenant un polypeptide pour former un complexe marqué.

**9.** Méthode selon la revendication 7 dans laquelle avant ledit ajout en mélange (a) ledit polypeptide est fixé à une matrice solide.

**10.** Système de diagnostic en kit pour l'analyse de la présence d'anticorps anti-HIV-1 dans un échantillon de fluide corporel comprenant un emballage comprenant un polypeptide ayant une séquence résidu d'amino-acide représentée par la formule :
LGZWGCSGKHIC,
dans laquelle Z est soit l'isoleucine soit la méthionine.

**11.** Système de diagnostic en kit pour analyse de la présence d'anticorps anti-HIV dans un échantillon de fluide corporel comprenant un emballage contenant au moins deux polypeptides dans lequel un premier desdits polypeptides a une séquence de résidu d'amino-acide représentée par la formule
LGZWGCSGKHIC,
dans laquelle Z est soit l'isoleucine soit la méthionine, et un second desdits polypeptides est capable d'immunoréaction avec des anticorps induits par un virus d'immunodéficience humaine.

**12.** Système de diagnostic selon la revendication 11 dans lequel ledit second desdits polypeptides a une séquence résidu d'amino-acide représentée par une formule sélectionnée dans le groupe consistant en :
LGLWGCSGKLIC,
LGIWGCSGKLIC, et
NSWGCAFRQVC.

**13.** Système de diagnostic selon la revendication 11 dans lequel lesdits premier et second polypeptides sont ajoutés en mélange.

**14.** Système de diagnostic selon la revendication 11 incluant de plus, dans un emballage séparé, un agent liant spécifique marqué pour signaler la présence d'un produit d'immunoréaction contenant un polypep-tide.

20

**15.** Système de diagnostic selon la revendication 11 dans lequel ledit polypeptide est fixé à une matrice solide.

**16.** Système de diagnostic en kit comprenant :

(a) un premier support solide composé d'une matrice solide ayant fixé opérationnellement à celle-ci un polypeptide ayant une séquence résidu d'amino acide représentée par la formule :
LGZWGCSGKHIC,
dans laquelle Z est soit l'isoleucine soit la méthionine, et
(b) un second support solide composé d'une matrice solide ayant fixée opérationnellement celle-ci un polypeptide ayant une séquence résidu d'amino-acide représentée par une formule sélectionnée dans le groupe consistant en :
LGLWGCSGKLIC,
LGIWGCSGKLIC, et
NSWGCAFRQVC.

**17.** Système de diagnostic en kit comprenant :

(a) un premier support solide composé d'une matrice solide ayant fixée opérationnellement à celle-ci un ajout en mélange d'au moins deux polypeptides dans lequel un premier desdits polypeptides a une séquence résidu d'amino-acide représentée par la formule :
LGZWGCSGKHIC,
dans laquelle Z est soit l'isoleucine soit la méthionine, et un second desdits polypeptides a une séquence résidu d'amino-acide représentée par une formule sélectionnée dans le groupe consistant en :
LGLWGCSGKLIC, et
LGIWGCSGKLIC; et
(b) un second support solide composé d'une matrice solide ayant fixée opérationnellement à celle-ci un polypeptide ayant une séquence résidu d'amino-acide représentée par la formule :
NSWGCAFRQVC.